(19) Europäisches Patentamt
European Patent Office
Office européen des brevets

(11) Veröffentlichungsnummer: **0 000 719**
**B1**

(12) # EUROPÄISCHE PATENTSCHRIFT

(45) Veröffentlichungstag der Patentschrift: **20.05.81**

(21) Anmeldenummer: **78100472.6**

(22) Anmeldetag: **21.07.78**

(51) Int. Cl.³: **C 07 D 493/08,** C 07 C 69/16, C 07 C 35/18, C 11 B 9/00, A 23 L 1/226 // (C07D493/08, 307/00)

(54) **Neue 1,4-Epoxy-trimethyl-butenyliden-cyclohexane (I), Verfahren zu deren Herstellung, Verwendung von (I) als Riech- und/oder Geschmackstoffe, Riech- und/oder Geschmackstoffkompositionen mit einem Gehalt an den neuen Cyclohexanen.**

(30) Priorität **25.07.77 LU 77834**
**19.05.78 CH 5464/78**

(43) Veröffentlichungstag der Anmeldung: **21.02.79 Patentblatt 79/4**

(45) Bekanntmachung des Hinweises auf die Patenterteilung: **20.05.81 Patentblatt 81/20**

(84) Benannte Vertragsstaaten: **CH DE FR GB NL**

(56) Entgegenhaltungen:
**CHEMICAL ABSTRACTS 49, (1955), 16 358c**
**HELVETICA CHIMICA ACTA, 42, (1959), Seiten 2557—2570**
**HELVETICA CHIMICA ACTA, 61, (1978), Seiten 373—382, publiziert Januar 25. 1978**

(73) Patentinhaber: **L. Givaudan & Cie Société Anonyme, Patentdienst Postfach, CH-4002 Basel (CH)**

(72) Erfinder: **Kaiser, Roman, Weidstrasse 6, CH-8610 Uster (CH)**

(74) Vertreter: **Lederer, Franz, Dr. et al, Patentanwälte Lederer, Meyer Lucile-Grahn-Strasse 22, D-8000 München 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchgebühr entrichtet worden ist (Art. 99(1) Europäisches Patentübereinkommen).



BUNDESDRUCKEREI BERLIN

# Neue 1,4-Epoxy-trimethyl-butenyliden-cyclohexane (I), Verfahren zu deren Herstellung, Verwendung von (I) als Riech- und/oder Geschmackstoffe, Riech- und/oder Geschmackstoffkompositionen mit einem Gehalt an den neuen Cyclohexanen

Die Erfindung betrifft neue Riech- und/oder Geschmackstoffe. Es handelt sich dabei um die Verbindungen der Formel

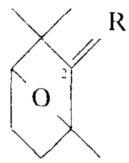

(I)

worin R den 2-cis- oder den 2-trans-Buten-1-ylidenrest bedeutet.

Die Formel umfaßt demgemäß die Verbindungen der Formel

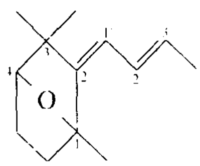

(Ia)

cis/trans

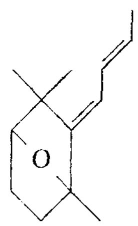

(Ib)

trans/trans

-1,4-Epoxy-1,3,3-trimethyl-2-(2-buten-1-yliden)-cyclohexan

Die Formeln sollen außerdem je beide der im Hinblick auf die vorhandene cis/trans-Isomerie (2'-Stellung) möglichen geometrischen Isomeren umfassen.

Die Erfindung betrifft ferner ein Verfahren zur Herstellung der Verbindungen der Formel I.

Dieses Verfahren ist dadurch gekennzeichnet, daß man 1,3,3-Trimethyl-2-(3-hydroxybutyliden)-6-cyclohexen-4-ol in saurem Medium zur Reaktion bringt.

Das saure Medium kann unter Zuhilfenahme von Mineralsäuren, wie Schwefelsäure oder Salzsäure, von sauren Salzen, wie Bisulfaten, beispielsweise $KHSO_4$, oder sauren Erden, wie Diatomeenerde, z. B. Filtrol, erzeugt werden. Aber auch geeignete organische Säuren, wie mittelstarke bis starke solcher Säuren kommen in Frage. Beispiele hierfür sind Alkansulfonsäuren, wie Methansulfonsäure, p-Toluolsulfonsäure oder Pikrinsäure.

Die Reaktionstemperatur ist nicht kritisch. Sie beträgt zweckmäßigerweise 20—200°C, vorzugsweise 60—130°C. Sie liegt zweckmäßigerweise höher bei Verwendung schwächerer Säuren und niedriger bei Verwendung stärkerer Säuren.

Die Umsetzung wird vorzugsweise unter Zuhilfenahme eines organischen Lösungsmittels, insbesondere eines aromatischen Lösungsmittels, durchgeführt. Beispiele sind Benzol, Toluol und Xylol.

Nach dem erfindungsgemäßen Verfahren fällt ein Gemisch von Ia : Ib im ungefähren Verhältnis 15 : 85 an. Im Gemisch können ferner Spuren des cis/cis- und des trans/cis-Isomeren nachgewiesen werden. Die Trennung des Isomerengemisches kann auf übliche Weise, z. B. mittels Säulenchromatographie oder präparativer Gaschromatographie erfolgen. Wie weiter unten ersichtlich, unterscheiden sich die Isomeren in ihren organoleptischen Eigenschaften nicht grundlegend, sodaß aus wirtschaftlichen Gründen das Isomerengemisch verwendet werden kann.

Das — weiter unten und im Beispiel 1 — als Verbindung 14 bezeichnete 1,3,3-Trimethyl 2-(3-hydroxybutyliden)-6-cyclohexen-4-ol stellt eine neue Verbindung dar Es kann — wie dort gezeigt — aus dem bekannten 2-Hydroxy-$\beta$-ionon erhalten werden.

Zu diesem Zweck wird dieses 2-Hydroxy-$\beta$-ionon zweckmäßigerweise mit einem Enolacylat in Anwesenheit einer der oben angegebenen Säuren umgesetzt; also z. B. in (einem Überschuß von) Acetonenolacetat gelöst, mit einer katalytischen Menge p-Toluolsulfonsäure versetzt und das Reaktionsgemisch während 1—2 Stunden bei Rückflußtemperatur gehalten. Das nach Aufarbeitung erhaltene Diacetat 13 kann durch Behandlung mit einem starken Reduktionsmittel, wie Lithiumaluminiumhydrid, direkt in das Diol 14 übergeführt werden.

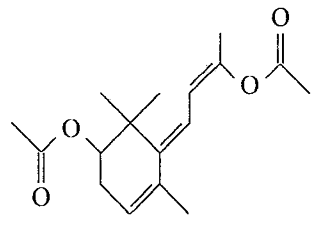

13

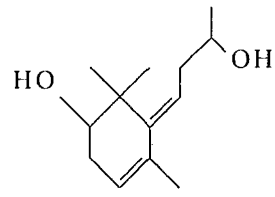

14

Die Verbindung 13 ist neu.

Die Verbindungen I weisen besondere organoleptische Eigenschaften auf, auf Grund derer sie sich vorzüglich als Riech- und/oder Geschmackstoffe eignen.

Die Erfindung betrifft demgemäß auch die Verwendung der Verbindungen I, insbesondere in praktisch reiner Form oder in Form von Gemischen (mit Ausnahme von natürlichen, I enthaltenden Gemischen) als Riech- und/oder Geschmackstoffe.

Unter praktisch reinen I sollen insbesondere solche I verstanden werden, die frei von Begleitstoffen sind, wie sie neben I in natürlichen Extrakten vorliegen. Als Praktisch reine I im Sinne der vorliegenden Erfindung sollen insbesondere synthetisch hergestellte I verstanden werden.

Die natürlichen, I enthaltenden Gemische sollen darum ausgeschlossen sein, da im Zuge der vorliegenden Arbeiten gefunden wurde, daß Ia/Ib, und zwar im Verhältnis von 1 : 5 im Osmanthus absolue enthalten sind.

Die erfindungsgemäß als Riech- und/oder Geschmackstoffe verwendeten Verbindungen I — insbesondere Ib [wegen seiner größeren Diffusion und Intensität] — zeichnen sich durch frische, grüne, würzige, sehr natürlich wirkende, an gewisse Aspekte von Cassisknospen und exotische Früchte — wie Mango, Passionsfrucht, Guanabana — erinnernde Geruchsnuancen aus. Die Verbindungen können demgemäß beispielsweise zur Parfümierung bzw. Aromatisierung von Produkten, wie Kosmetika (Seifen, Saleben, Pudern, Zahnpasten, Mundwässern, Desodorantien, Shampoos, Lotionen, etc.), Detergentien, bzw. Nahrungsmitteln, Genußmitteln und Getränken dienen, wobei die Verbindungen vorzugsweise nicht allein, sondern in Form von Kompositionen mit andern Riech- bzw. Geschmackstoffen eingesetzt werden. Solche Riech- bzw. Geschmackstoffkompositionen mit einem Gehalt an Verbindungen I und deren auf an sich bekannte Art erfolgende Herstellung (Zugabe von I zu bekannten Riech- bzw. Geschmackstoffkompositionen oder Vermischung von I mit als Bestandteil von Riech- bzw. Geschmackstoffkompositionen geeigneten natürlichen oder synthetischen Verbindungen oder Gemischen) bildet ebenfalls Gegenstand der Erfindung.

Als Riechstoffe eignen sich die Verbindungen I, insbesondere Ib, auf Grund ihrer oben beschriebenen, originellen Noten, insbesondere in Kombination mit einer Reihe von natürlichen und synthetischen Riechstoffen wie z. B.

- Galbanumöl, Mastixöl, Vetiveröl, Patchouliöl, Sandelholzöl, Mandarinenöl, Petitgrainöl, Ylang-Ylang-Öl, Basilikumöl, Baummoos absolue, Patchouliblätteröl, Cedernöl, Fichtenöl, Lorbeeröl, Costuswurzelöl, Calmusöl, Beifußöl, Kamillenöl, Wermutöl, Wurmsamenöl, Selleriesamenöl, Angelikasamenöl, Sternanisöl, Thymianöl, Rosmarinöl, Lavendelöl, Lavandinöl, Aspiköl, Salbeiöl, Neroliöl, Bergamottöl, Citronenöl, Orangenöl, Grapefruitöl, Geraniumöl, Benzoeresinoid, Melilotus absolue, Jasmin absolue, Rosenöl, Canangaöl, Corianderöl, Cassie absolue, Narzissen absolue, Verveine absolue oder Öl, Veilchenblätter absolue, Tuberosen absolue usw.
- Aldehyden wie Hydroxycitronellal, Zyklamenaldehyd, p-tert.-Butyl-$\alpha$-methylhydrozimtaldehyd, alpha-Hexylzimtaldehyd, 3,5-Dimethyl-cyclohex-3-en-1-yl-carboxaldehyd, Citral, Citronellal, 2,6-Dimethyl-6-hepten-1-al, Isovaleraldehyd, trans-2-Hexenal, Sorbinaldehyd, trans-2-Octenal, n-Octanal, n-Nonanal, trans-2, -cis-6-Nonadienal, 2,4-Decadienal, Methylnonyl-acetaldehyd usw.
- Ketonen wie alpha-Ionon, beta-Ionon, Allylionon, Acetanisol, 4-(para-Hydroxyphenyl)-2-butanon, Campher, Menthon, Carvon, Pulegon usw.
- Acetalen und Ketalen wie Phenylacetaldehyd-dimethylacetal, Phenylacetaldehyd-glycerinacetal, 2-Methyl-1,3-dioxolan-2-äthylacetat, Capronaldehyd-dimethylacetal usw.
- Äthern wie Eugenolmethyläther, Methyl-1-methylcyclododecyläther, Anethol, Estragol usw.
- Phenolkörpern wie Eugenol, Isoeugenol, Creosol usw.
- Alkoholen wie Butanol, cis-3-Hexanol, trans-2,cis-6-Nonadienol, cis-6-Noneol, Linalool, Geraniol, Nerol, Citronellol, Nerolidol, Farnesol, Benzylalkohol, Phenyläthylalkohol, Zimtalkohol usw.
- Estern wie Methyldihydrojasmonat, Linalylacetat, Geranylacetat, Cedrylacetat, Vetiverylacetat, Äthylisovalerat, Äthylcaproat, para-tert.-Butylcyclohexylacetat, ortho-tert.-Butylcyclohexylacetat, Myraldylacetat[R] (Givaudan), Benzylacetat, Benzylsalicylat, Styrallylacetat, Äthyl-$\alpha$-methyl-phenylglycidat, Äthyl-trans-2-hexenoat, Äthyl-trans-2-octenoat usw.
- Lactone wie $\gamma$-Undecalacton, $\gamma$-Decalacton, $\gamma$-Nonalacton, $\delta$-Decalacton, $\delta$-Octalacton, Cumarin usw.
- Säuren wie Milchsäure, Buttersäure, $\alpha$-Methylbuttersäure, trans-2-Hexensäure, trans-2-Octensäure usw.

- Moschus- und ambraartig riechenden Körpern wie Äthylenbrassylat, 4-Acetyl-6-tert.-butyl-1,1-dimethylindan, 12-Oxahexadecanolid, 8α,12-Oxido-13,14,15,16-tetranorlabdan usw.
- schwefelhaltigen Verbindungen wie p-Menthan-8-thiol-3-on, Dimethylsulfid und anderen Sulfiden und Disulfiden usw.
- stickstoffhaltigen Verbindungen wie Methylanthranilat, Indol, Isobutylchinolin, verschiedenen Pyrazinen, 5-Methyl-heptan-3-on-oxim usw.

Die unter Verwendung von Verbindungen I hergestellten Riechstoffkompositionen, insbesondere vom Typ Chypre, Cologne, Muguet bzw. allgemein blumiger und holziger Richtung bestechen insbesondere durch ihre eindrückliche Frische und Originalität.

Bei der Herstellung solcher Kompositionen können die oben aufgeführten bekannten Riechstoffe nach (dem Parfumeur bekannter) Art und Weise verwendet werden, wie z. B. aus W. A. Poucher, Perfumes, Cosmetics and Soaps 2, 7. Auflage, Chapman und Hall, London, 1974 hervorgehend.

Es hat sich schließlich gezeigt, daß die Verbindungen Ia/Ib auch bei der Rekonstitution einer Reihe von ätherischen Ölen oder Absolues, wie z. B. Mandarinenöl, Grapefruitöl, Cassis-Knospen absolue — in denen diese Verbindungen nie nachgewiesen wurden — verwendet werden können.

Die Konzentration der Verbindungen I kann je nach dem Verwendungszweck innerhalb weiter Grenzen variieren, beispielsweise zwischen etwa 0,01 (Detergentien) und etwa 15 Gew.-% (alkoholische Lösungen). In Parfumbasen bzw. Konzentraten können die Konzentrationen selbstverständlich auch höher liegen. Die Parfumbasen können in üblicher Weise zur Parfümierung von Eaux de Cologne, Eaux de toilette, Lotionen, Crèmes, Shampoos, Seifen und Detergentien, etc. verwendet werden.

Als Geschmackstoffe können die Verbindungen I beispielsweise zur Erzeugung bzw. Verbesserung, Verstärkung, Steigerung oder Modifizierung von Frucht- oder Beerenaromen in Nahrungsmitteln (Joghurt, Süßwaren, etc.); in Genußmitteln (Tee, etc.) und Getränken (Limonaden etc.) verwendet werden.

Die ausgeprägten geschmacklichen Qualitäten von insbesondere praktisch reinen, insbesondere von synthetisch hergestellten Verbindungen I ermöglichen die Verwendung in geringen Konzentrationen. Eine geeignete Dosierung umfaßt beispielsweise den Bereich von 0,1 ppm – 100 ppm, vorzugsweise von 1 ppm – 20 ppm im Fertigprodukt, d. h. dem aromatisierten Nahrungsmittel, Genußmittel oder Getränk.

In der folgenden Tabelle sind einige Effekte zusammengestellt, wie sie sich mit den Äthern I erzielen lassen.

Tabelle

| Aroma | Dosierung | Effekt |
|---|---|---|
| Passionsfrucht | ppm im Fertigprodukt<br>0,1–30 ppm<br>insb. 0,5–4 ppm | größere Natürlichkeit des Fruchtcharakters, volles Aroma |
| Grapefruit | ppm im Fertigprodukt<br>0,1–100 ppm<br>insb. 1–20 ppm | sehr natürlicher Fruchtcharakter |
| Mango | ppm im Fertigprodukt<br>0,1–50 ppm<br>insb. 0,5–10 ppm | unterstützt den Charakter der frischen, reifen Mangofrucht |

Die Verbindungen können auf übliche Weise mit den für Geschmackstoffkompositionen verwendeten Bestandteilen vermischt bzw. solchen Aromen zugesetzt werden. Unter den erfindungsgemäß verwendeten Aromen werden Geschmackstoffkompositionen verstanden, die sich auf an sich bekannte Art verdünnen bzw. in eßbaren Materialien verteilen lassen. Sie können nach an sich bekannten Methoden in die üblichen Gebrauchsformen, wie Lösungen, Pasten oder Pulver übergeführt werden. Die Produkte können sprühgetrocknet, vakuumgetrocknet oder lyophilisiert werden.

Die bei der Herstellung solcher Aromen zweckmäßigerweise verwendeten bekannten Aromastoffe sind entweder in der obigen Zusammenstellung bereits enthalten oder können leicht der Literatur entnommen werden, wie z. B. J. Merory, Food Flavorings, Composition, Manufacture and Use, Second Edition, The Avi Pbublishing Company, Inc., Westport, Conn. 1968, oder G. Fenaroli, Fenaroli's Handbook of Flavor Ingredients, Second Edition, Volume 2, CRC-Press, Inc. Cleveland, Ohio, 1975.

Spektrale Daten:

5 IR: 3460, 1730, 1336, 1151, 1049, 1039, 1029 $cm^{-1}$;
NMR: 0,92+0,98 (je 3H,s); 1,25 (3H,t,J ~ 6,8 Hz); 1,62 (3H); 2,76 (1H); 4,0 (1H,m); 4,08 (2H,q,J ~ 6,8 Hz); 5,4 (m,1H) δppm;
MS: 212 ($M^+$, 9); 194 (25), 166 (28), 141 (19), 139 (27), 123 (28), 121 (100), 113 (32), 107 (18), 95 (53), 43 (27)

6 IR: 3460, 1720, 1280, 1235, 1030 $cm^{-1}$;
NMR: 1,12 (6H,2s); 1,30 (3H,t,J ~ 6,8 Hz); 1,67 (3H,s); 3,52 (1H,m); 4,20 (2H,q,J ~ 6,8 Hz) δppm;
MS: 212 ($M^+$, 6); 169 (69), 139 (55), 125 (42), 123 (81), 121 (42), 96 (63), 95 (68), 79 (40), 67 (35), 55 (43), 43 (100)

7 IR: 1730, 1310, 1216, 1179, 1119, 1046, 1000, 871 $cm^{-1}$;
NMR: 0,98+1,21 (6H,2s); 1,24 (3H,t,J ~ 6,8 Hz); 1,45 (3H,s); 2,29 (1H); 3,90 (1H,d,J ~ 4 Hz); 4,11 (2H,q,J ~ 6,8 Hz) δppm
MS: 212 ($M^+$, 15), 197 (45), 167 (64), 154 (76), 139 (50), 130 (84), 121 (65), 109 (36), 95 (52), 83 (44), 55 (39), 43 (100).

Das obige Rohprodukt (32,0 g) — gelöst in 80 ml Tetrahydrofuran — ließ man im Verlaufe von 30 Minuten zu einer Suspension von 4,60 g (0,12 Mol) Lithiumaluminiumhydrid in 300 ml wasserfreiem Tetrahydrofuran zutropfen. Man rührte das entstandene Gemisch während 16 Stunden bei Rückflußtemperatur. Das auf 0° abgekühlte Reaktionsgemisch wurde vorsichtig mit Wasser, hierauf mit 2 N Salzsäurelösung versetzt, in 400 ml Äther aufgenommen und die ätherische Schicht mit Wasser gewaschen, getrocknet und eingeengt. Es verblieben 26,2 g Rohprodukt, welches gemäß gaschromatographischer Analyse 80% 2-Hydroxy-α-cyclogeraniol 8 enthielten.

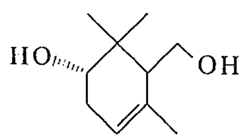

8

Eine aus Äther umkristallisierte Probe zeigte die folgenden spektroskopischen Daten:

IR: 3615, 3450, 1175, 1068, 1037, 1018, 955, 845 $cm^{-1}$ (als Lösung in $CHCl_3$);
NMR: 0,90+1,09 (je 3H,2s); 1,75 (3H); 3,80 (2H,d,J ~ 4 Hz); 3,95 (1H,m); 5,50 (1H,m) δppm;
MS: 170 ($M^+$, 1), 152 (83), 121 (80), 109 (36), 107 (52), 95 (47), 93 (34), 81 (79), 72 (59), 55 (42), 43 (100), 41 (51);
Smp. 103 – 104°.

15,0 g (0,088 Mol) rohes 2-Hydroxy-α-cyclogeraniol 8 wurden in 60 ml Methylchlorid gelöst und im Verlaufe von 10 Minuten so zu einer Lösung von 19,0 g (0,088 Mol) Pyridiniumchlorochromat (E. J. Corey, J. W. Suggs, Tet. Lett. 31. 2647 [1975]) in 250 ml Methylchlorid zugetropft, daß die Reaktionstemperatur zwischen 20 und 25° blieb. Anschließend rührte man noch während 20 Minuten bei Raumtemperatur, dekantierte die Reaktionslösung vom gebildeten Niederschlag ab, spülte den Niederschlag zweimal mit Methylenchlorid und wusch die kombinierten organischen Phasen mit Wasser, 2 N Salzsäure, Bicarbonatlösung nochmals mit Wasser. Nach dem Trocknen und Einengen verblieben 11,9 g Rohprodukt, welche die beiden Hydroxyaldehyde

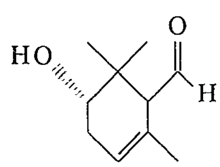

9

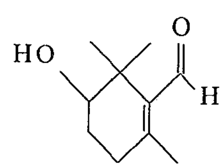

10

im Verhältnis von 3 : 1 enthielten.
Spektrale Daten:

9 MS: 168 ($M^+$, 23), 150 (14), 137 (38), 121 (55), 107 (42), 97 (59), 95 (44), 79 (28), 72 (30), 55 (40), 43 (10);
10 MS: 168 ($M^+$, 16), 137 (100), 123 (15), 109 (31), 95 (11), 81 (46), 79 (14), 70 (78), 67 (31), 55 (13), 41 (21)

Für die Herstellung solcher üblicher Gebrauchsformen kommen beispielsweise folgende Trägermaterialien, Verdickungsmittel, Geschmackstoffverbesserer, Gewürze und Hilfsingredientien in Frage:

Gummi arabicum, Tragant, Salze oder Brauereihefe, Alginate, Carrageen oder ähnliche Absorbentien; Indole, Maltol, Gewürzoleoresine, Raucharomen; Gewürznelken, Natriumcitrat; Mononatriumglutamat, Dinatrium-inosin-5'-monophosphat (IMP), Dinatriumguanosin-5-phosphat (GMP); oder spezielle Aromastoffe, Wasser, Äthanol, Propylenglykol, Glycerin.

Beispiel 1

Eine Lösung von 25,0 g (0,129 Mol) $\alpha$-Safransäureäthylester 2 und 0,25 g Bengalrosa in 300 ml Äthanol wurde in einer Belichtungsapparatur aus Pyrexglas bei gleichzeitigem Durchperlen von reinem Sauerstoff während 2 Stunden belichtet. Als Lichtquelle diente ein zentralangeordneter, wassergekühlter Quecksilber-Hochdruckstrahler Typ TQ-150 der Firma Hanau. Die mittlere Sauerstoffaufnahme betrug rund 30 ml/Minute. Der Reaktionsverlauf wurde auf gaschromatographischem Wege (Abnahme des Eduktes) verfolgt.

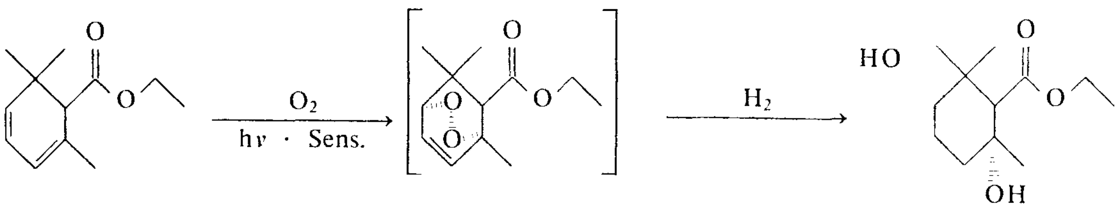


2 3 4

Die so erhaltene ähtanolische Lösung des 1,4-Epidioxy-1,3,3-trimethyl-2-äthoxycarbonyl-5-cyclohexens 3 wurde mit 0,3 g Platin(IV)-oxyd versetzt und anschließend bei Normaldruck bis zur Sättigung hydriert. Der Wasserstoffverbrauch betrug 4,95 l in 1,5 Stunden (≙77,5% der Theorie). Die Reaktionslösung wurde durch Filtration vom Katalysator befreit, eingeengt und die erhaltenen rohen 31 g Reaktionsprodukte an der 20fachen Menge Kieselgel chromatographiert. Die Elution mit Äther ergab 14,0 g cis-2,5-Dihydroxy-2,6,6-trimethylcyclohexancarbonsäureäthylester 4 vom Schmelzpunkt 92–94° (Ausbeute = 51%, Reinheit > 98%).

IR: 3600, 3450, 1720, 1333, 1142, 1031, 1025, 932, 891 $cm^{-1}$ (als Lsg. in $CHCl_3$);
NMR: 1,02 + 1,18 (je 3H,s); 1,23 (3H,s); 1,28 (3H,t,J ~ 6,8 Hz); 2,57 (1H,s); 3,90 (1H,m,$J_{ax\text{-}ax}$ ~ 8 Hz, $J_{ax\text{-}eq}$ ~ 4 Hz); 4,15 (2H,q, J ~ 6,8 Hz)$\delta$ppm;
MS: 212 ($M^+$ – $H_2O$, 13), 166 (34), 154 (15), 129 (100), 123 (25), 113 (15), 101 (68), 95 (24), 83 (43), 43 (90).

34,9 g (0,152 Mol) des Dihydroxyesters 4 und 3,0 g p-Toluolsulfonsäure wurden in 250 ml Toluol gelöst und anschließend während 2 Stunden bei gleichzeitiger Entfernung des Reaktionswassers bei der Rückflußtemperatur des Reaktionsgemisches gerührt. Die abgekühlte Reaktionslösung verdünnte man mit 200 ml Äther, wusch je dreimal mit Sodalösung und Wasser, trocknete mit Natriumsulfat und engte ein. Es resultierten 32,0 g Rohprodukt, welche gemäß gaschromatographischer Analyse 83% bzw. 10% der 2-Hydroxy-$\alpha$- und $\beta$-cyclogeranium-säureester 5 und 6 enthielten. Als Nebenprodukt entstand zu 7% der 2,5-Oxaester 7.

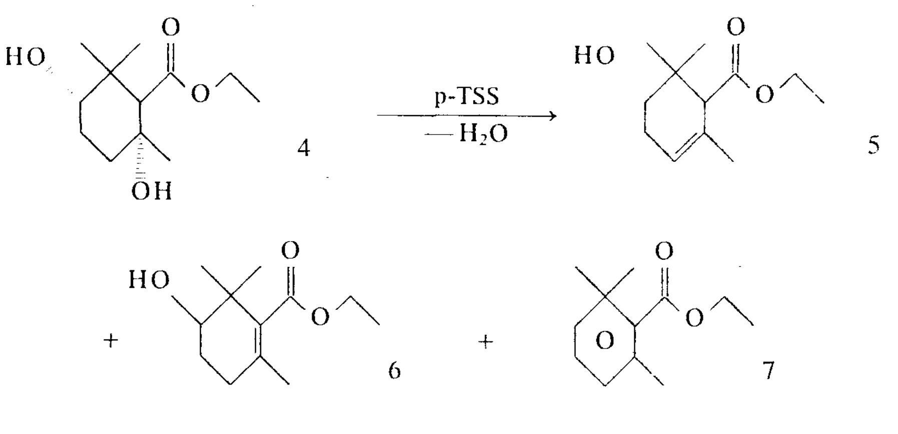


4 5

+ 6 + 7

11,9 g des rohen Aldehydgemisches 9/10 wurden in 120 ml Aceton gelöst, mit 23 ml 10%iger wäßriger KOH-Lösung versetzt und anschließend während 19 Stunden bei Rückflußtemperatur gerührt. Die Hälfte des Acetons wurde unter vermindertem Druck abdestilliert, das Konzentrat in 200 ml Äther aufgenommen und die ätherische Lösung mit konzentrierter Kochsalzlösung gewaschen. Nach Trocknen und Einengen verblieben 12,5 g Rohprodukt, welche gemäß gaschromatographischer Analyse zu rund 40% aus den 2-Hydroxy-$\beta$-iononen 11 und 12 im Verhältnis von 1 : 5 bestanden. Die säulenchromatographische Reinigung des Rohproduktes lieferte bei der Elution mit Hexan/Äther = 1 : 1 4,2 g 92%iges Gemisch der 2-Hydroxy-ionone 11 und 12.

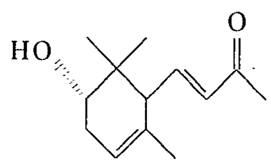

11

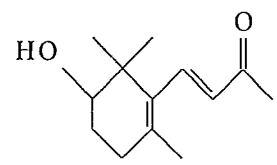

12

Zur spektroskopischen Charakterisierung gelangten Proben, welche mit Hilfe von präparativer Gaschromatographie auf eine Reinheit von > 95% gebracht wurden.
Spektrale Daten:

11 IR: 3450, 1670, 1620, 1256, 1051, 989, 905, 815 $cm^{-1}$;
NMR: 0,96 (6H,2s); 1,58 (3H); 2,27 (3H,s); 2,54 (1H,d,J ~ 10 Hz); 3,68 (1H,dxd, $J_{ax\text{-}ax}$ ~ 7 Hz, $J_{ax\text{-}eq}$ ~ 5 Hz); 5,44 (1H,m): 6,08 (1H,d,J ~ 16 Hz); 6,68 (1H,dxd, $J_1$ ~ 16 Hz), $J_2$ ~ 10 Hz) $\delta$ppm;
MS: 208 ($M^+$, 1); 175 (51), 157 (10), 147 (19), 137 (26), 121 (43), 109 (13), 93 (51), 77 (14), 72 (21), 43 (100);
12 IR: 3460, 1665, 1608, 1256, 1190, 1175, 1115, 1042, 1005, 978 $cm^{-1}$;
NMR: 1,08 + 1,11 (je 3H,2s); 1,75 (3H); 2,29 (3H,s); 3,56 (1H,dxd, $J_{ax\text{-}ax}$ ~ 7 Hz, $J_{ax\text{-}eq}$ ~ 4,6 Hz), 6,10 (1H,d,J ~ 16,5 Hz); 7,21 (1H,d,J ~ 16,5 Hz) $\delta$ppm;
MS: 208 ($M^+$, 2), 193 (69), 175 (44), 157 (8), 149 (26), 147 (20), 121 (36), 105 (33), 83 (16), 81 (19), 79 (14), 43 (100).

3,87 g der 2-Hydroxy-ionone 11 + 12 (1 : 5) wurden in 20 ml Acetonenolactat gelöst und anschließend in Gegenwart von 0,03 g p-Toluolsulfonsäure 2 Stunden bei Rückflußtemperatur gerührt. Das Reaktionsgemisch wurde abgekühlt, in 150 ml Äther aufgenommen, die ähterische Phase mit Bicarbonatlösung und Wasser gewaschen, getrocknet und eingeengt. Es verblieben 4,5 g des 4-Acetoxy-1,3,3-trimethyl-2-(3-acetoxy-2-butenyliden)-cyclohex-6-ens 13, welche wie folgt direkt zum Diol 14 weiterverarbeitet wurden:

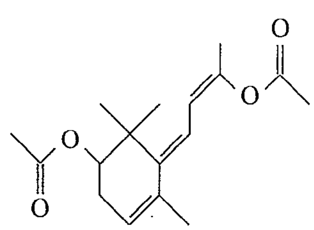

13

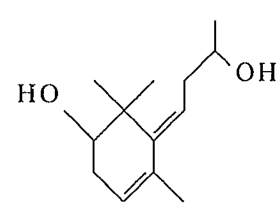

14

Eine Lösung von 3,9 g Diacetat 13 in 10 ml Äther ließ man im Verlaufe von 10 Minuten zu einer Suspension von 1,06 g Lithiumaluminiumhydrid in 70 ml Äther zutropfen, rührte das Reaktionsgemisch während einer Stunde bei Rückflußtemperatur, kühlte hierauf auf Raumtemperatur, zersetzte den Überschuß an Lithiumaluminiumhydrid vorsichtig mit Wasser, wusch die Ätherphase mit Wasser, trocknete und engte ein. Die verbliebenen 2,7 g Diol 14 löst man zusammen mit 0,05 g p-Toluolsulfonsäure in 25 ml Benzol und rührte das Gemisch anschließend während einer Stunde bei Rückflußtemperatur, verdünnte das abgekühlte Gemisch mit 50 ml Äther und wusch die Ätherphase mit Bicarbonatlösung und Wasser. Nach dem Trocknen und Einengen verblieben 2,3 g Rohprodukt, welche gemäß gaschromatographischer Analyse neben schwererflüchtigen Verbindungen zu rund 50% aus 1,4-Epoxy-1,3,3-trimethyl-2-(2-buten-1-yliden)-cyclohexan 1a und 1b (Verhältnis cis/trans = 15 : 85) bestand.

Durch Kugelrohrdestillation des Rohproduktes erhielt man 0,95 g Produkt, welches zu über 92% aus dem Isomerengemisch bestand. Das Gemisch besitzt einen frischen, grünen, würzigen, sehr natürlich wirkenden Geruch, der an gewisse Aspekte von Tomatenblättern, Cassisknospen und exotischen Früchten erinnert.

Zur spektroskopischen Charakterisierung gelangten Proben der einzelnen Isomeren, welche durch Chromatographie an der 40fachen Menge Kieselgel (Hexan/Äther = 30 : 1) auf eine Reinheit von über 98% gebracht worden waren.
Spektrale Daten:

1a IR: 1410, 1183, 1118, 1019, 1002, 990, 961, 928, 878, 833, 770 $cm^{-1}$;
NMR: 1,04 + 1,13 (je 3H,s); 1,75 (3H,s); 1,78 (3H,d,J ~ 7 Hz); 3,93 (1H,d,J ~ 4 Hz); 5,55 (1H,dxq,$J_{3',2'}$ ~ 16 Hz, $J_{3'}$, $CH_3$ ~ 7 Hz), ~ 5,55 (1H,d,J ~ 10 Hz), ~ 6,3 (1H,dxd, $J_{2',3'}$ ~ 16 Hz, $J_{2',1'}$ ~ 10 Hz);
MS: 192 ($M^+$, 24); 149 (16), 136 (22), 123 (45), 121 (35), 109 (49), 93 (26), 91 (32), 81 (44), 69 (86), 43 (100)
1b IR: 1421, 1235, 1195, 1117, 1021, 1003, 963, 872, 823 $cm^{-1}$;
NMR: 1,28 (6H,2s); 1,50 (3H,s); 1,78 (3H,d,J ~ 7 Hz); 3,89 (1H,d,J ~ 4 Hz); 5,55 (1H,dxq, $J_{3',2'}$ ~ 16 Hz; $J_{3',CH_3}$ ~ 7 Hz); 5,65 (1H,d,$J_{1',2'}$ ~ 10 Hz); 6,30 (1H,dxd,$J_{2',3'}$ ~ 16 Hz; $J_{2',1'}$ ~ 10 Hz);
MS: 192 ($M^+$, 24), 149 (12), 136 (12), 123 (39), 121 (22), 109 (36), 93 (18), 91 (18), 81 (33), 69 (82), 43 (100).

## Beispiel 2

### Grün-Base

| | Gewichtsteile |
|---|---|
| Methyldihydrojasmonat | 400 |
| Bergamottöl | 200 |
| Propylenglykol | 145 |
| Allylionon | 50 |
| Fixateur 404® (8α,12-Oxido-13,14,15,16-tetranorlabdan) | 5 |
| | 800 |

Gibt man zu dieser konventionellen Grünbase (Richtung Salbei) 200 Teile der neuen Verbindung I*), wird die Base in Richtung Tomatenblätter und Cassis-Knospen sehr angenehm abgerundet. Die neue Substanz fügt sich sehr harmonisch in den Komplex ein und verleiht ihm eine frische, würzige etwas trockene und viel weniger süße Note.

## Beispiel 3

### Parfumerie-Komposition (Richtung Grapefruit, Zitronenschale)

| | Gewichtsteile |
|---|---|
| Bergamottöl | 300 |
| Mandarinenöl | 150 |
| Galbanumöl | 100 |
| Äthylenbrassylat® Givaudan | 50 |
| p-Methan-8-thiol-3-on | 1 |
| Lösungsmittel ad | 900 |

Werden 100 Teile der neuen Verbindung I zu dieser Base mit schwarzem Johannisbeer-Charakter gegeben, verschwindet die Cassis-Note zu Gunsten einer sehr angenehm frischen Note Richtung Rapefruit und Zitronenschale. Die Substanz verbindet sich, wie ersichtlich, sehr harmonisch mit schwefelhaltigen Verbindungen. Die neue Base erhält eine sehr natürliche Frische. Überraschenderweise wird dieser Frische-Effekt auch nach 24 Stunden noch sehr deutlich wahrgenommen.

*) Unter »Verbindung I« wird in den Formulierungsbeispielen das im Beispiel 1 erhaltene Isomerengemisch verstanden.

Beispiel 4

Parfumerie-Komposition (Grünnote)

| | Gewichtsteile |
|---|---|
| Linalylacetat | 300 |
| α-Hexylzimtaldehyd | 300 |
| Benzylsalicylat | 300 |
| Methyldihydrojasmonat | 30 |
| Methylanthranilat | 10 |
| Basilikumöl | 10 |
| Cyclal®Givaudan(3,5-Dimethyl-cyclohex-3-en-1-carboxaldehyd) (10% in Propylenglykol) | 5 |
| Galbanumöl | 5 |
| | 960 |

Gibt man zu dieser Grün-Base 40 Teile der neuen Verbindung I, so wirkt die Komposition viel diffusiver, frischer, natürlicher. Die Grün-Note erhält einen fruchtigen Aspekt Richtung Cassis-Knospen. Der — für die Grünnote wesentliche — Komplex von Galbanumöl und Methyldihydrojasmonat wird durch Zugabe von I in äußerst vorteilhafter Weise unterstrichen.

Beispiel 5

Parfümerie-Komposition (maskuline Chypre-Note)

| | Gewichtsteile |
|---|---|
| Bergamottöl | 300 |
| Citronellol | 160 |
| Patchouliöl | 100 |
| Vetiveröl | 100 |
| Hydroxycitronellal | 80 |
| Eugenol extra | 70 |
| Baummoos absolue | 40 |
| Geraniol | 40 |
| Methyleugenol | 30 |
| Styrallylacetat | 20 |
| Sandelholzöl | 20 |
| Methylnonylacetaldehyd (10% in Propylenglykol) | 10 |
| Methyldihydrojasmonat | 8 |
| Phenylacetaldehyddimethylacetal | 2 |
| | 980 |

Versetzt man diese Komposition (Chypre-Charakter) mit 20 Teilen der neuen Verbindung I, so wirkt die gesamte Komposition lebhafter, wirkt gleichzeitig abgerundeter, hat eindeutig mehr Charakter eines »fertigen« Cologne. Die neue Komposition eignet sich gut zur Herstellung eines maskulinen Herren-Cologne.

Beispiel 6

Parfümerie-Komposition (Muguet-Charakter)

| | Gewichtsteile |
|---|---|
| Hydroxycitronellal | 440 |
| Rhodinol | 340 |
| Linalool | 90 |
| α-Amylzimtaldehyd | 50 |
| Sandelholzöl | 50 |
| Ylang-Ylang-Öl Bourbon | 20 |
| | 990 |

Versetzt man diese konventionelle Muguet-Komposition mit 10 Teilen der neuen Verbindung I, so wird sofort eine eindeutige Verbesserung des Geruchseindrucks in Richtung der natürlichen Vorlage festgestellt.

Gewisse Aspekte des taufrischen Maiglöckchens kommen nun sehr schön zur Geltung.

Beispiel 7

1 Liter eines handelsüblichen Passionsfrucht-Saftes wurde mit 0,35 g einer 0,1%igen alkoholischen Lösung des 1,4-Epoxy-1,3,3-trimethyl-2-(2-butenyliden)-cyclohexanes versetzt. Entsprechend versetzte man 1 Liter Passionsfrucht-Saft mit 0,35 g reinem Alkohol. Die beiden Fruchtsäfte wurden einer Gruppe von 10 Testpersonen zur sensorischen Beurteilung vorgelegt. Die Mehrheit der Testpersonen (8) erklärte, daß in der zusätzlich aromatisierten Probe der angenehm süße, exotisch wirkende Geruchsaspekt der Passionsfrucht eindeutig besser zur Geltung käme.

Beispiel 8

Analog dem vorgehenden Beispiel wurde 1 Liter Grapefruit-Saft mit 0,40 g einer 0,1%igen alkoholischen Lösung des 1,4-Epoxy-1,3,3-trimethyl-2-(2-butenyliden)-cyclohexanes versetzt. In diesem Falle wurde im Vergleich zur Blindprobe von der Mehrheit der Testpersonen (8) eine ausgeprägtere Holznote festgestellt, zudem wurde eine Assoziation an reife Grapefruit-Früchte äußerst positiv vermerkt.

Beispiel 9

1 kg Yoghurt »nature« wurde mit 100 g Mango-Fruchtpulpe und 80 g Saccharose versetzt. Zu 500 g dieses Mango-Yoghurts gab man 0,25 g einer 0,1%igen alkoholischen Lösung des 1,4-Epoxy-1,3,3-trimethyl-2-(2-butenyliden)-cyclohexanes.

Die beiden Yoghurts wurden dem Testpanel (10 Personen) vorgelegt und im Vergleich zu einer frischen Mangofrucht sensorisch beurteilt.

Die Mehrheit der Testpersonen (6) fand im zusätzlich mit I aromatisierten Yoghurt eindeutig wesentlich mehr Charakter der frischen Mango-Frucht.

**Patentansprüche**

1. Verbindungen der Formel

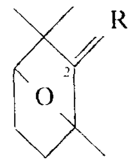

(I)

worin R den 2-cis- oder den 2-trans-Buten-1-ylidenrest bedeutet.

2. cis/trans-1,4-Epoxy-1,3,3-trimethyl-2-(2-buten-1-yliden)-cyclohexan gemäß Anspruch 1 der Formel

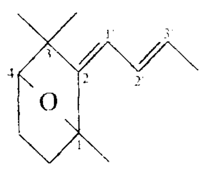

(Ia)

3. trans/trans-1,4-Epoxy-1,3,3-trimethyl-2-(2-buten-1-yliden)-cyclohexan gemäß Anspruch 1 der Formel

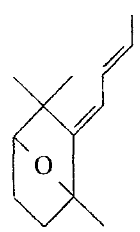

(Ib)

4. Praktisch reine Verbindungen der Formel I gemäß Anspruch 1.
5. Synthetisch hergestellte Verbindungen der Formel I gemäß Anspruch 1.
6. Verfahren zur Herstellung der Verbindungen der Formel I gemäß Anspruch 1, dadurch gekennzeichnet, daß man 1,3,3-Trimethyl-2-(3-hydroxybutyliden)-6-cyclohexen-4-ol in saurem Medium zur Reaktion bringt.
7. Riech- und/oder Geschmackstoffkompositionen, gekennzeichnet durch einen Gehalt an einer Verbindung der Formel I gemäß Anspruch 1 in praktisch reiner Form oder in Form von Gemischen, mit Ausnahme der natürlichen, Verbindungen der Formel I enthaltenden Gemische.
8. Riech- und/oder Geschmackstoffkompositionen nach Anspruch 7, dadurch gekennzeichnet, daß die Verbindungen der Formel I synthetisch hergestellt worden sind.
9. Verwendung von Verbindungen der Formel I gemäß Anspruch 1 in praktisch reiner Form oder in Form von Gemischen, mit Ausnahme der natürlichen, Verbindungen der Formel I enthaltenden Gemische, als Riech- und/oder Geschmackstoffe.
10. Verwendung von Verbindungen der Formel I gemäß Anspruch 1 nach Anspruch 9, dadurch gekennzeichnet, daß die Verbindungen der Formel I synthetisch hergestellt worden sind.

**Claims**

1. Compounds of the general formula

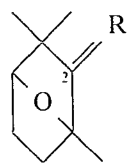

(I)

wherein R represents the 2-cis- or the 2-trans-buten-1-ylidene group.
2. Cis/trans-1,4-epoxy-1,3,3-trimethyl-2-(2-buten-1-ylidene)-cyclohexane according to cleim 1 of the formula

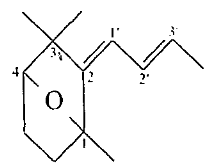

(Ia)

3. Trans/trans-1,4-epoxy-1,3,3-trimethyl-2-(2-buten-1-ylidene)-cyclohexane according to claim 1 of the formula

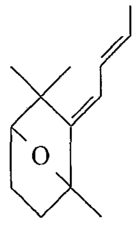

(Ib)

4. Practically pure compounds of the general formula I according to claim 1.
5. Synthetically manufactured compounds of the general formula I according to claim 1.
6. A process for the manufacture of the compounds of formula I given in claim 1, which process comprises reacting 1,3,3-trimethyl-2-(3-hydroxy-butylidene)-6-cyclohexen-4-ol in acid medium.
7. Odorant and/or flavouring compositions which contain a compound of formula I given in claim 1 in practically pure form or in the form of mixtures, with the exception of natural mixtures containing compounds of formula I.
8. Odorant and/or flavouring compositions according to claim 7 which contain synthetically manufactured compounds of formula I.
9. The use of compounds of formula I given in claim 1 in practically pure form or in the form of mixtures, with the exception of natural mixtures containing compounds of formula I, as odorant and/or flavouring compositions.
10. The use according to claim 9 of compounds of formula I given in claim 1, characterized in that the compounds of formula I have been synthetically manufactured.

**Revendications**

1. Composés de formule

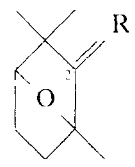

(I)

dans laquelle R représente le radical 2-cis- ou 2-trans-butén-1-ylidène.

2. Le cis/trans-1,4-époxy-1,3,3-triméthyl-2-(2-butén-1-ylidène)-cyclohexane selon la revendication 1 de formule

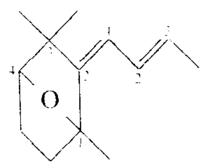

(Ia)

3. Le trans/trans-1,4-époxy-1,3,3-triméthyl-2-(2-butén-1-ylidène)-cyclohexane selon la revendication 1 de formule

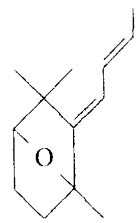

(Ib)

4. Composés pratiquement purs de formule I selon la revendication 1.

5. Composés, préparés synthétiquement, de formule I selon la revendication 1.

6. Procédé pour la préparation de composés de formule I selon la revendication 1, caractérisé par le fait qu'on fait réagir le 1,3,3-triméthyl-2-(3-hydroxybutylidène)-6-cyclohexén-4-ol en milieu acide.

7. Compositions de substances odoriférantes et/ou aromatisantes, caractérisées par le fait qu'elles contiennent un composé de formule I selon la revendication 1, à l'état pratiquement pur ou à l'état de mélanges, à l'exception des mélanges naturels contenant des composés de formule I.

8. Compositions de substances odoriférantes et/ou aromatisantes suivant la revendication 7, caractérisées par le fait qu'elles contiennent un composé de formule I préparé synthétiquement.

9. Utilisation, comme substances odoriférantes et/ou aromatisantes, des composés de formule I selon la revendication 1 à l'état pratiquement pur ou à l'état de mélanges, à l'exception des mélanges naturels contenant des composés de formule I.

10. Utilisation selon la revendication 9 de composés de formule I selon la revendication 1 préparés synthétiquement.